# EUROPEAN PATENT SPECIFICATION

(11) **EP 1 131 408 B1**
(45) Date of publication and mention of the grant of the patent: **29.03.2006**
(21) Application number: 99954269.9
(22) Date of filing: 16.11.1999
(51) Int. Cl.: C12N 5/06

(54) **CELLS, CULTURE METHODS, AND THEIR USE IN AUTOLOGOUS TRANSPLANTATION THERAPY**
ZELLEN, KULTURVERFAHREN, UND IHRE VERWENDUNG IN AUTOLOGER TRANSPLANTATIONSTHERAPIE
CELLULES, PROCEDES DE CULTURE ET LEURS UTILISATIONS

(30) Priority: 16.11.1998 GB 9825096
(43) Date of publication of application: 12.09.2001
(73) Proprietor: Hebe Limited, Hamilton HM 12 (BM)
(72) Inventor: DAVIES, Alison, Bridgend, Mid Glamorgan CF32 0ND (GB)
(74) Representative: Towler, Philip Dean
(86) International application number: PCT/GB1999/003813
(87) International publication number: WO 2000/029551

(56) References cited:
- EP-A- 0 668 013
- WO-A-94/16715
- WO-A-97/30157
- WO-A-98/39427
- US-A- 5 750 376

## Description

The present invention relates to autologous transplantation therapy and in particular to the removal of samples of eukaryotic tissues or cells from a healthy host organism for subsequent transplantation to that host, after a temporal change to the host, for example when the need arises, e.g. a therapeutic need. The advantages are that cells held in suspended animation (ie. dormant cells) can be manipulated and/or revitalised at a future date when required eg. for therapy. Cell samples in a state of suspended animation can also be accumulated by performing several rounds of harvesting of primary samples from the same source organism(s) prior to the manipulation and/or revitalization.

The maintenance and replication of eukaryotic cells in culture has been practised for many years. Studies at the beginning of this century (Proc. Soc. Exp. Biol. Med. 4 (1907) 140; J. Exp. Med. 15 (1912) 516) have demonstrated that it is possible to remove animal or human tissue samples and maintain the cells therefrom in *in vitro* culture for various lengths of time depending upon the culture conditions. Most early culturing consisted of immersing animal tissue or cells in blood, or blood components such as serum. Blood or serum was the major component of the medium within which tissue/cell samples were cultured. However, as our knowledge of the *in vitro* requirements of cells has increased, the use of serum or blood components in cell/tissue culture medium has decreased to the extent where fully defined media are now available which provide all the nutrients and supplements necessary to maintain at least some cell types in culture (see e.g. Freshney's Tissue Culture of Animal Cells, (Culture of Animal Cells: A Manual of Basic Technique, Wiley Liss, 1994)).

However, the maintenance of eukaryotic cells in culture for sustained periods has always been and remains fraught with difficulty. The major problem is that it is generally not possible to keep eukaryotic cells taken from multicellular organisms in primary culture for more than a few days to weeks. This is because cells in primary culture have a limited lifespan. In some instances, though, their maintenance can be prolonged indefinitely. For example, a single cell, or group of cells, can undergo genetic changes which enable it/them to maintain continuous cell replication in an *in vitro* culture environment. Such genetic changes usually involve mutations which activate cellular proto-oncogenes to become oncogenes, and/or mutations which restrict or negate the activity of tumour suppressor genes, leading to the loss of replication inhibition and to the development of cellular immortality (Trends in Genetics 9(1993)138). Our current understanding implies that tumour cells give rise to cancers not because of the sudden activation of immortalizing oncogenes, but because of mutations in genes which normally regulate the cell's ability to limit its own replication. These genetic events, which occur *in vivo* as well as *in vitro*, have led to the generation from multicellular organisms of eukaryotic cell lines that can be maintained in continuous culture (Culture of Animal Cells: A Manual of Basic Technique, Wiley Liss, 1994).

Although it is possible to maintain cell lines in culture, their ability to undergo continuous replication may make it disadvantageous or undesirable to do so. In order to save on resources, it would be better if it were possible to store cells until they were required for culture. Technologies permitting such storage have been developed, with much information coming from studies with prokaryotic organisms.

Early work with prokaryotic organisms such as bacteria and viruses showed that it was possible to keep them in a state of dormancy for long periods of time without affecting their ability to survive and replicate once revived, or revitalised, from their state of dormancy. It was shown that prokaryotic organisms could be put into a state of dormancy (suspended animation) using a number of methods such as freezing, freeze-drying, drying or by placing them in various organic or inorganic solutions with or without subsequent freezing. The solutions include dimethylsulphoxide (DMSO), ethanol, ether, glycerol, phosphate buffered sodium chloride, and serum, or mixtures thereof, or with any other substance that can prolong shelf-life but is not confined to them.

Many, if not all, of the methods for placing or maintaining prokaryotic cells in a state of dormancy have also been applied to eukaryotic cells.

Maintaining cells in a culture environment enables manipulations to be performed on cells *in vitro*, and this advantage has led to the development of cell-based assays in diagnostic technology. Cell culturing, therefore, either prior to inducing dormancy or after cell revitalisation has been shown to have important applications for diagnostic medicine as well as basic science (Bone 22(1998)7; J Bone Min Res 13(1998)432).

In addition to medical diagnosis, cell culture methods have also been applied to medical therapies. For example, in cases where patients are suffering from leukaemia, one approach to alleviate the disease is to eradicate the patient's tumour cells by radiotherapy, chemotherapy and/or surgery. However, radiotherapy and chemotherapy, which are seemingly the only practical treatments for diseases which are systemic and/or metastatic, may also destroy or substantially deplete the patient's normal, non-tumour haematopoietic cells. Consequently, it is standard practice to replace the patient's depleted normal cells with those from the bone marrow of a donor. The donor is often a close relative whose 'tissue-type' is similar to that of the patient, and the donor tissue is therefore less likely to be rejected by the patient (Adv Immunol 40(1987)379).

In addition to possible rejection of the grafted cells by the host, there is also the potential problem of graft versus host (GVH) disease. The vast majority of lymphocytes in a marrow donor sample are immature and unable to elicit a full-blown immune response without first undergoing a process of maturation. Maturation occurs when lymphocytes are processed via the thymus. If immature lymphocytes from the donor are processed through the new host's thymus they will accept the host as "self". However, it is inevitable that a proportion of the donor T-lymphocytes will have undergone maturation via the donor's thymus prior to transplantation, and, consequently, might regard the recipient as foreign. If so, the mature donor T-lymphocytes may attempt to attack the host's cells leading to GVH disease (Immunol Rev 157(1997)79).

To reduce the possibility of the graft rejecting the host, the donor marrow samples can be trawled with, for example, antibodies which specifically recognise and bind to mature T-cells allowing for their removal or lysis prior to transplantation (Curr Op Oncol 9(1997)131). It can be seen, therefore, that the in vitro culture of donor human cells and their manipulation prior to grafting is a recognized methodology in transplantation therapy.

For the treatment of diseases such as leukaemias and lymphomas it is often more practical to provide donor marrow well before it is required for transplantation to the patient. In these instances, the donor marrow sample is made dormant e.g. by the addition of DMSO to the sample followed by freezing of the sample. The donor sample may be kept in a frozen state for, potentially, many years prior to its use for grafting, with little deterioration. Moreover, the donor cells may be manipulated, eg. the mature T-lymphocytes removed, either before or after freezing. The ability to store the marrow samples for long periods has enabled donor marrow banks to be set up to support treatment programmes for patients with various leukaemias and lymphomas (Bone Marrow Transpl 17(1996)197).

The recognition that it was mature T-lymphocytes in donor marrow that caused GVH disease, and the development of technologies to effectively remove them from donor marrow, has helped make significant advances in bone marrow allografting.

By way of definition, allograft means cells or tissue grafted or transplanted between different members of the same species; a xenograft is a transplant of tissue/cells between members of different species; and an autograft is a tissue/cell graft from self to self.

Prior to the scientific advances which made allografting feasible for the treatment of lymphoma and leukaemia, bone marrow transplantation was restricted to autografting (Stem Cells 13(Suppl 3) (1995) 63) . As defined above, autografting is where cells/tissue are removed from an individual, and grafted back to the same individual. Autografting remains commonplace, and is particularly relevant in the treatment of burns where skin is removed from undamaged regions of the body and grafted to help repair/regenerate the damaged skin areas (Burns 24(1998)46). Autografting is also common in orthopaedic surgery where the patient's own bone is taken from eg. the pelvis, rib, or chin and used to augment/repair bone in another region of the body, eg. the face (J Oral Maxillo Surg 54(1996)420).

Autografting for the treatment of leukaemias and lymphomas has advantages and disadvantages. The key advantage to the patient is that there is no problem of rejection (either by the patient or by the graft) when cells/tissue from the patient are returned to the patient. The main disadvantage, though, is that the grafted cells/tissue removed for subsequent grafting may contain diseased cells. The value of autografting, therefore, is dependent on the ability to obtain or produce donor tissue which is disease-free.

Leukaemias and lymphomas, by definition, are diseases affecting cells of the blood and the lymph, and the medical consensus is that seeding or infiltrating of diseased cells to bone marrow can occur irregularly, may involve specific bone marrow sites, and/or happen late in the onset disease. Hence, the rationale for autografting leukaemia/lymphoma patients is that, once the patient has been treated by radiotherapy and/or chemotherapy to destroy tumour cells, it should be possible to return their own, essentially disease-free, bone marrow.

To further ensure that the autografted sample is essentially free of disease, it can be treated in a number of ways. For example, it can be purged by separating/destroying residual tumour cells in the sample. A common purging method, for instance, is to apply tumour cell-identifying antibodies to tag the tumour cells in the sample - the tumour cells can then be removed using cell-sorting technology such as fluorescence-activated cell sorting (Curr Op Hematol 4(1997)423).

The value of autografting for the treatment of metastatic or systemic disease such as leukaemia and lymphoma remains questionable, though, since the donor sample may still contain some element of the disease which cannot be completely purged with current technologies. The quality of the autograft will also depend on the status of the disease in the donor material eg. the type and aggressive nature of the cells involved, the ability of diseased cells to seed the marrow, and the time from onset of the disease when the donor sample was taken. In essence then, the value of an autograft in such circumstances is empirical and will vary significantly between individual patients who present with various symptoms of proliferative disease.

Advances in therapy continue to be made, and our greater understanding of disease processes helps us to modify and refocus our therapeutic approaches to alleviate disease and suffering. Such understanding has been greatly advanced by technological improvements in the field of molecular biology. We are now in a position to follow the pathogenesis of diseases at a molecular level, and recognize the importance of an individual's genetic make-up in predisposing them to certain diseases. For example, we are aware that some individuals, because of their genetic composition, are prone to cancers, e.g. leukaemias and vascular disorders such as heart disease. They may be also predisposed to neurodegenerative diseases such as Alzheimer's disease and Huntington's chorea, as well as to endocrine and exocrine diseases such as diabetes and hypothyroidism, and skeletal disorders such as age-related osteopaenia, osteoporosis, arthritis and periodontal disease. Through genetic testing, therefore, it is now possible to identify those individuals predisposed to debilitating diseases.

Furthermore, our knowledge of the body's immune system, and in particular the way in which it recognises and kills virally-infected and tumour cells, continues to advance. We now know that in order to elicit cell-mediated immunity, an offending cell (e.g. a virally-infected or tumour cell) must co-present an HLA class I restricted tumour or viral epitope with danger signals such as GM-CSF and/or TNF-α, so that the antigen-presenting cells (APC) of the immune system will express co-stimulatory signals such as B7 and IL-12 in conjunction with antigen to the interacting cytotoxic T-lymphocyte (CTL) population. The co-presentation leads to the production of clones of both activated and memory cells (for review see Nature Medicine Vaccine Supplement 4(1998)525). In the absence of these additional signals, HLA-I antigen-restricted T-cells which recognise offending cells are processed for destruction or desensitization (a bodily process presumably put into place to avoid the development of eg. autoimmune disease). The induction of such tolerance is because of either ignorance, anergy or physical deletion (Cold-Spring Harbour Symp Quant Biol 2(1989)807; Nature 342 (1989) 564; Cell 65 (1991) 305; Nature Med 4(1998)525). It is now clear that tumour cells do not automatically co-present danger and/or co-stimulatory signals. Hence, the spawning of a tumour may lead to eradication of the very cells that provide cell-mediated immunity against the tumour. A patient presenting with a cancer, leukaemia/lymphoma or sarcoma etc, therefore, may have already removed their innate ability to destroy the tumour, by default. However, if the required T-lymphocytes, or a sample thereof, were removed from the patient prior to the onset of proliferative disease, the relevant T-cell population could now be returned to the patient, after the necessary co-stimulation of the T-cells, so as to alleviate disease.

The present invention is based on our recognition of this possibility, namely the concept of removing cells or tissues from a healthy host organism for subsequent transplantation to that same host organism in a subsequent autologous (autogeneic) transplantation procedure, when the need or desire to do so arises. In one aspect, the present invention thus provides use of a host cell population of mature, healthy lymphocyte cells obtained from blood of a non-diseased host organism for the preparation of a cell composition for use in subsequent autologous therapeutic transplantation therapy of a disease or disorder of said host organism, wherein the cells are obtained from the host organism before the disease or disorder develops or manifests itself.

Also disclosed is a method of autologous transplantation therapy, said method comprising obtaining a population of host cells from a non-diseased host organism; and preparing a cell composition from said host cell population for subsequent transplantation to said host organism.

The specification also discloses a cell composition comprising a host cell population obtained from a non-diseased host organism for use in subsequent autologous transplantation therapy of said host organism.

The host organism may be any eukaryotic organism, but preferably will be an animal, more preferably a mammal, and most preferably a human. Other representative host organisms include rats, mice, pigs, dogs, cats, sheep, horses and cattle.

The term "non-diseased" is used herein to describe a state in which the host organism is not suffering from, or demonstrating symptoms of, the disease or disorder, which it is subsequently intended to treat by the transplantation, procedure.

Furthermore, in certain embodiments of the invention, the host organism is preferably not predisposed to, or at risk from, any particular disease or disorder e.g. preferably not exhibiting any symptoms or manifestations predictive of a subsequent disease or disorder. Likewise, the host organism is preferably not suffering from any injuries or damage which may give rise to an anticipated or expected condition. A major idea or concept behind the present invention is to harvest or collect the host cells from the host organism at a stage when there is no direct prediction, suggestion, or suspicion that a particular disorder or disease may develop, for use against a future possible or unpredicted event, or an event which may occur simply by chance, rather than an anticipated or suspected or predicted illness or condition.

Also included, is the removal of cells from a "non-diseased" region or area of the body of the host organism, even though other areas or regions of the body or cells or tissues of the body may be affected by a disease or disorder. What is required is that the cells removed are themselves healthy ie. "non-diseased" within the definition given above.

The cells are obtained from the host organism before any disease or disorder develops or manifests itself, and more preferably when the host organism is in general good health, and preferably not immunocompromised in any way.

Advantageously, therefore, the host cells are obtained from host organisms when they are young, preferably in adolescence or early adulthood- In the case of humans, cell sampling at the ages of about 12 to 30, preferably 15 to 25 is preferred. Especially preferably, sampling is from the age of 16 or 17 upwards, for example in the age range 16 to 30, 17 to 30, or 18 to 30, or perhaps 18 to 35 or 40. It is thus preferred that the cells be obtained when the host organism is mature, or reaching maturity, but before the processes of ageing or senescence have significantly set in. In particular, it is preferred and advantageous that the immune system of the host organism is mature or fully developed. However, the obtention of cells outside these ranges is encompassed, and cells may be obtained at any post-natal life stage e.g. from juvenile host organisms e.g. in mid-to late childhood, or even infants, or from older individuals, as long as they remain "non-diseased". Foetal host organisms are thus excluded from the present invention and sampling of foetal cells is not encompassed.

In contrast to sampling umbilical blood for example, the advantages of the present invention are that taking cells from post-natal or older hosts allows multiple samples to be collected, thereby increasing the opportunity of storing sufficient number of cells. In addition sampling from juvenile or older hosts overcomes the ethical requirements such as providing informed consent.

Sampling from adolescent or adult host organisms is preferred since the sampled cells, from blood in particular, will contain a greater proportion of valuable mature T-cells capable of recognising aberrant cell populations, such as cancer cells or virally-infected cells. Thus, when blood samples are used, it is advantageous that they are taken from an individual with a mature immune system (ie. not foetal or neonatal).

The term "autologous" is used herein to mean that the transplantation is to the same organism (ie. the same individual) from which the host cells were removed. Thus, autogeneic transplantation [self-to-self] or autografting is intended.

"Transplantation" refers to any procedure involving the introduction of cells to an organism. Thus, any form of transplantation or grafting known in the art is encompassed.

"Transplantation therapy" refers to any procedure involving transplantation of cells. Both therapeutic (e.g. curative or palliative, or symptom-relieving) and prophylactic (ie. preventative or protective) therapies are covered. Thus, the term "transplantation therapy" encompasses the transplantation of cells to a host organism in need thereof, or in anticipated, expected or suspected need thereof, for any reason. Advantageously, the transplantation therapy is to treat or alleviate a disease or disorder which develops, or is threatened, subsequently, e.g. cancer or infection, or a degenerative condition (e.g. neuro-degenerative).

The term "host organism" as used herein means the post-natal body, and does not include "waste" or "disposable" tissues which are not part of the body per se. Thus, the umbilical cord and placenta are not included. However, any part of the actual body of the host organism may be used as the source of the host cells.

A preferred lymphocyte cell type according to the invention is the T-lymphocyte (a T-cell). Mature T-lymphocytes are particularly preferred.

The disease or disorder which may be treated by the transplantation therapy may be any disease or disorder known to man. Thus any disease condition, illness, disorder or abnormality of the body is included. Mention may be made, for example, of infections e.g. diseases arising from pathogenic activity e.g. bacterial, fungal or viral infections, or infections by any other organism e.g. a protozoa or other parasite; any malignant or pre-malignant condition; proliferative or hyper-proliferative conditions; or any disease or diseases arising or deriving from or associated with a functional or other disturbance or abnormality, in the cells or tissues of the body, e.g. aberrant gene expression or cell or tissue damage (whether induced or caused by internal or external causes e.g. ageing, injury, trauma or infection etc.), or idiopathic diseases (e.g. Parkinson's disease).

Advantageously, the present invention has particular utility in the therapy of chronic conditions (ie. chronic diseases or disorders).

Representative diseases or disorders thus include any cancer (whether of solid tissues of the body (e.g. prostate or manmary tumours), or of haemopoietic tissues or other individual cells, in particular leukaemias or lymphomas), vascular disorders, neural disorders including in particular neuro-degenerative conditions, endocrine and exocrine diseases and skeletal disorders, as discussed above, or any condition associated with ageing or senescence. Particular mention may be made of osteoporosis and osteoarthritis.

Uses of the invention for the therapy of cancer represents a preferred embodiment of the invention, and includes cancers of any cells or tissues of the body. The invention is not limited to any one type of cancer (e.g. leukaemia, lymphoma, carcinoma or sarcoma), nor is it restricted to specific oncogenes or tumour-suppressor gene epitopes such as ras, myc, myb, fos, fas, retinoblastoma, p53 etc. or other tumour cell marker epitopes that are presented in an HLA class I antigen restricted fashion. All cancers such as breast, stomach, colon, rectal, lung, liver, uterine, testicular, ovarian, prostate and brain tumours such as gliomas, astrocytomas and neuroblastomas, sarcomas such as rhabdomyosarcomas and fibrosarcomas are included for the therapy by the present invention.

A further preferred embodiment of the invention is the use of the invention for the therapy of infections, particularly viral infections, including HIV and other infections which may have a latent phase.

The host cell population which is used according to the invention for a subsequent transplantation procedure to the host organism, may be used at any convenient or desired time after removal from the host organism. In order words a tissue or cell sample removed from an individual may be used at a future date when required for use in therapy. Advantageously, however, the invention permits the subsequent transplantation to be at a prolonged time interval after the cell removal, e.g. from 3 months to many years (e.g. up to 80 years or more). Thus, the invention allows healthy, non-diseased cells to be removed from an individual when in good health, or good immunological status and used, many years later, for therapy of that individual, when a problem develops. Preferred or representative time intervals for subsequent transplantation thus include 6 months to 70 years, 1 to 50 years, and 1 to 30 years or 5 to 30 years. Conventional cryopreservation conditions and procedures allow for such periods (Scand. J. Haematol. 10 (1977) 470; Int. J. Soc. Exp. Haematol. 7 (1979) 113).

The host cell population may be obtained or removed from the host cell organism in any convenient way. This may depend on the cells and the location in the body from which they are obtained.

Recent advances have been made in the way cells may be obtained for subsequent grafting. The advent of molecular biology has helped us to understand more clearly the basic biology of cell growth and function in health and disease. For example, investigations into the agents which regulate haematopoiesis have led to the isolation of a series of factors that influence the proliferation and differentiation of lymphocytes - these include the cytokines such as the interleukin series IL-1-IL-18 and the leukotrienes; and growth factors such as the TNF's, the TGF's, FGF's, EGP's, GM-CSF, G-CSF and others. A number of these factors are now available commercially for clinical use, and some have been shown to increase substantially the number of lymphocytic cells and, in particular, immature T-lymphocytes in the peripheral blood. Their administration to the host organism means that, after a few days to allow an effect, it is possible to filter large quantities of the cells of interest directly from host's blood without the need to sample the marrow (Stem Cells 15(1997)9). The technology for extracting lymphocytes from blood, by removing blood from the patient, passing it through a cell separator and then returning it to the patient, all virtually simultaneously, has been available for many years (Practical Immunology, 3rd Edition, Blackwell Scientific Publications, 1989).

The removed host cell population may be stored, cultured, handled, manipulated or treated in any known or desired manner for subsequent transplantation (i.e. to prepare the cell composition for transplantation). Cell handling, culture and storage procedures are well known in the art and widely described in the literature, and any of the standard procedures may be used. Cells may be stored in any convenient or desired medium, e.g. as known in the art. (See e.g. Freshney's (supra) for cell culture requirements and WO 98/33891 for lymphocyte preparation).

Conveniently, the host cell population may be put into a state of dormancy. The term ''dormancy" as used herein includes any state of suspended animation or stasis, and procedures for achieving this are well known in the art, as described above. Any of the known procedures may be used (see e.g. Freshneys, supra). Thus, the cells may be held or maintained in a quiescent, inactive or non-proliferating state.

According to a preferred procedure, the cells are frozen preferably to a temperature below -160°C.

A particularly preferred means of achieving dormancy is to freeze the cells to the boiling point of helium (He) ie. to about -269°C or below.

Thus, in a further aspect, the present invention provides a method of making and/or maintaining lymphocyte cells dormant, said method comprising feezing said cells to a temperature at or below -269°C.

As described in Freshneys (supra), the cells may be suspended in a suitable medium (e.g. containing 5-10% DMSO) and cooled at a controlled rate e.g. 1°C per minute to -70°C, then into liquid/gae N₂. Such conventional procedures may be adapted to cool the cells into He/N₂ mixtures or He.

Results have been obtained which show that by using the (ie. the lower temperature) improvements in the long-term viability of the cells may be obtained. When multiplied over 10-20 years for example, this enhancement in viability may be important in the successful storage of the cells (see Example 6 below).

Alternative methods of achieving and/or maintaining cell dormancy include cooling to 4°C.

The cells may be cultured if desired, for example as part of a treatment or modification process (see later) or they may be expanded ie. they may be cultured to increase cell numbers. For example, the cells may be passaged, according to methods well known in the art. The culturing may be before or after the period of dormancy, or both.

Prior to transplantation, the cells may also or alternatively be modified or manipulated in some way, e.g. genetically or functionally and/or by inducing or modulating their differentiation. Again, as described above, this is known in the art and any of the known or standard procedures may be used. (see e.g. WO98/06823, WO98/32840, WO98/18486). Such modification or manipulation may be carried out before or after dormancy, or both. The modification/manipulations are not restricted temporally, in that the sequence and/or number of manipulations is flexible.

Thus, genetic interventions may include regulating or modifying the expression of one or more genes, e.g. increasing or decreasing gene expression, inactivating or knocking out one or more genes, gene replacement, expression of one or more heterologous genes etc. The cells may also be used as a source of nuclei for nuclear transfer into stem cells.

The cells may be exposed to or contacted with factors, e.g. cytokines, growth factors etc. which may modify their growth and/or activity etc, or their state of differentiation etc. The cells may also be treated to separate or selectively isolate or enrich desired cell types or to purge unwanted cells.

Thus, for example a T-cell modificatory method is discussed above, whereby T-cells are co-stimulated prior to transplantation.

Alternatively, haematopoietic cells may be co-presented with HLA class I restricted tumour antigen and B7 and/or IL12, so as to produce both activated and memory T-cells. The sample may then returned to the host organism before the onset of disease, as a prophylactic therapy. Alternatively, the co-presenting antigen-presenting cells (APCs) may be returned to the host along with the activated and/or memory T cells. Alternatively, the cells may be exposed to the host's tumour *in vitro* with appropriate danger signals and co-presentation of co-stimulatory molecules, before being returned to the host. As with our WO96/15238 directed to T-lymphocyte therapy, the host's CTLs may be genetically modified to recognize the tumour prior to replacement. The alternatives in this paragraph provide for functional interactions between haematopoietic cells either prior to, or after a period of dormancy or combination.

Following dormancy, the cells are revitalised prior to use in transplantation. Again, this may be achieved in any convenient manner known in the art, and any method of revitalising or reviving the cells may be used.

Conveniently, this may, for example, be achieved by thawing and/or diluting the cells, e.g. as described in the Examples. Techniques for revitalisation are well known in the art (see e.g. Freshney's supra). Cells may be thawed by gentle agitation of the container holding the cells in water at 37°C, followed by dilution of DMSO to 1% or below, e.g. with medium, or patient serum etc. Cells may be implanted immediately or after recovery in culture. Revitalisation is designed to re-establish the usefulness of the cells e.g. in prophylaxis or curative therapy.

The invention relates to the recognition that a tissue sample from a non-diseased individual may be put into a state of dormancy. The tissue may then be revitalized and returned to the same individual when required at a later date. Grafting the revitalized tissue 1, 2, 3, 4, 5, 6 or more months or 1, 2, 3, 4, 5, 6 or more years after its removal from the patient is intended to alleviate or protect against disease, to slow the progression of disease, or to augment and/or support the functioning of the remaining normal, or damaged, tissue in the patient. The invention is clearly distinct from the freezing of bone marrow cells from patients with eg. leukaemia, and from the freezing of gametes, eg. sperm, prior to treatment of patients with eg. childhood leukaemia, because the patients already have disease. It is also distinguished from patients who provide blood for chilled storage for possible later use, eg. at a subsequent operation, for the same reason. In addition, the duration of storage for the possible return of such a blood sample is commonly only up to one month. Similarly, individuals with no diagnosed abnormality may choose to provide blood for chilled storage for prospective use by themselves prior to travelling abroad. Such use might include for the treatment of hypovolaemia after acute blood loss, such might occur after a road traffic accident or other trauma, but this again is for a short period of storage of about one month only, and not intended for use in future disease e.g. chronic disease.

A number of particularly advantageous applications of the invention can be identified. Firstly, for individuals who are predisposed to blood disorders such as leukaemia or lymphoma but have not succumbed to, or are asymptomatic for, the disease prior to sampling, the invention provides a prospective therapeutic method. It would be beneficial for such presently healthy individuals to provide a tissue sample or multiple tissue samples (eg. bone marrow or blood). The sample/s could be kept in a state of dormancy until their use at a future date to replace/augment aberrant or lost tissues/cells and alleviate the disease they were likely to contract after the sample was taken. The invention may also have applicability for individuals whose environments pre-dispose them to e.g. leukaemia, for example power station workers. The invention is against all conventional teachings, then, which recommend retrospective allografts or autografts to provide a curative intervention in diseases such as leukaemia and lymphoma or solid tumours. Other types of predisposition are also included within the scope of this aspect of the invention, as indeed are other factors e.g. a family history which might suggest a risk of a suspected condition.

A further advantageous utility of the invention is in the treatment of HIV infection or disease. Thus, CD4⁺ cells can be collected from an individual when healthy or non-infected, and stored for subsequent transplantation into said individual when HIV infection manifests itself or when AIDS develops, or CD4⁺ cell count falls etc. Such a procedure may be attractive to an individual with a life-style likely to place them at risk from contracting HIV infection.

In addition, it is well recognized that the ageing process makes individuals more susceptible to disease. The basis for the susceptibility appears to be in the loss of immune function resulting from a significant decrease in T and B cell numbers/activity during ageing (Mech Ageing & Dev 91(1996)219; Science 273(1996)70; Mech Ageing & Dev 96(1997)1).

Furthermore, the invention can be seen as being particularly advantageous in the light of recent discoveries related to the down-regulation of cytotoxic T-lymphocyte activity in response to HLA class I antigen-restricted tumour-epitope presentation.

Disease susceptibility is particularly pertinent when elderly patients are subjected to eg. surgery in a hospital environment, where they are prone to opportunistic infections with serious or even fatal consequences. Marrow and/or blood samples taken much earlier in life from the patient, such as during adolescence or early adulthood when their immune system is mature but uncompromised, and maintained subsequently in a state of dormancy, could be revitalized and reinfused to the patient to boost their immune system. Such an approach would provide for a method of augmenting the patient's immune system after surgery in order to lessen the likelihood of post-operative complications caused by opportunistic infections. The invention, therefore, could be used as a prophylactic therapy, eg- for elderly patients when they are more susceptible to disease.

Another area in which the invention can be seen to have particular advantages is where individuals may be predisposed to endocrine disorders in later life such as diabetes; hypothyroidism or hypoparathyroidism, or to the loss or disease of skeletal material leading to age-related osteopaenia, osteoporosis, osteoarthritis, rheumatoid arthritis, and periodontal disease. 4 Tissue/cell samples could be taken from these individuals, stored in a state of dormancy, and then reinfused back, optionally after *in vitro* expansion, into the individual when their endocrine/skeletal status indicated a requirement.

Furthermore, the invention has particular advantages in the treatment of neurodegenerative illness with a genetic component. This is because the donor cells can be modified genetically, either before or after dormancy to, for example, override, negate, alleviate or reverse the effects, future or current, of the abnormal inherited component of the disease. In Huntington's chorea, for example, the IT15 gene coding for huntingtin contains an abnormally large number of CAG repeats (Cell 72 (1993) 971). This dominant gene may be inactivated or knocked out *in vitro,* and replaced by the normal version (J. Neuro Sci: 18 (1998) 6207; Bioessays 20 (1998) 200). The present invention has clear advantages, therefore, in the treatment of neurodegenerative disease, by providing graftable (PNAS 89 (1992) 4187), and in this case autograftable material, both with and without prior modification of the cell's functionality, differentiation or genotype. Analogous principles would apply to the treatment of other diseases or disorders.

The invention would also have advantages where cell/tissue samples need to be transported to specialist laboratories to undergo manipulations (eg. genetic modifications e.g. nuclear transplantation into stem cells) prior to their return to the patient. Often, it may not be possible to treat/modify the cells, either genetically or functionally, or phenotypically at the place where the patient is sampled. Even if it is possible, the process may not be immediately initiatable. Placing the sample in a state of dormancy may be considerably advantageous to the procedure, as the cell manipulations that need to be made can be performed at a time suitable to the management of the process eg. either before making the cells dormant or after they are resuscitated, but before they are returned to the patient.

The invention would be seen also as advantageous when a multiplicity of samples from a single donor are needed. The invention could be used to build a stock by multiple sample additions (i.e. 2 or more) to the first sample, all of them being placed in a state of dormancy prior to revitalizing part of, or the complete collection for use, for example, in therapy. The donor tissue for autografting may be from animals including transgenic animals. Such animals would include, but not be limited to, rats, mice, pigs, dogs, cats, sheep horses and cattle.

The invention may also include the incorporation of a negative selection marker into all cells/tissues destined to be returned to the patient as described, for example, in WO96/14401 (Transgenic organisms and their uses),and WO96/14400 (Genetically modified neural cells).

The invention will now be described in more detail in the following non-limiting Examples, with reference to the drawings in which:

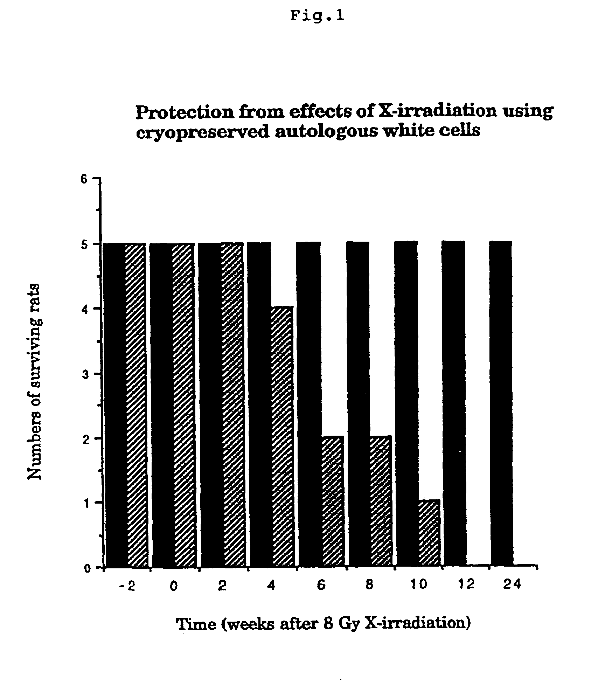

Figure 1 is a histogram showing the effect of reinfusion of cryopreserved autologous white cells on survival of X-irradiated rats (numbers of surviving rats vs Time (weeks) after irradiation). Rate were maintained under SPF (specific pathogen-free) conditions. Five ml of whole blood was removed by cardiac puncture from all rats two weeks prior to irradiation. White cells were prepared as described earlier from five of the blood samples and then cryopreserved using standard procedures (see Example 1). At time zero, all rats were given 8 Gy of X-irradiation. Two weeks following the irradiation five rats (solid bars) were infused with autologous grafts of thawed white cells and control animals (striped bars) received autologous grafts of thawed white cells and control animals (striped bars) received autologous plasma vehicle alone. Two days later, all rats were removed from SPP conditions and returned to the main animal housing facility. Death of animals through opportunistic infection was monitored. The experiment demonstrates that reinfusion of white cells into irradiated rats protects such immune-depleted animals from death by infection.

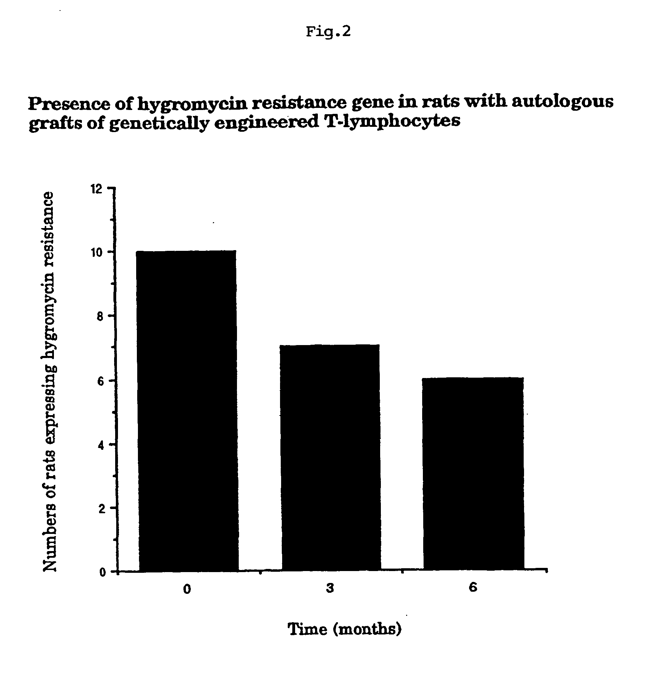

Figure 2 is a histogram showing maintenance in grafted rats of autologous, engineered T-lymphocytes up to six months after grafting, the full period of study (numbers of rats vs time (months)). Five ml of peripheral blood was removed from each of ten rats by cardiac puncture. The white cells were enriched from the samples as described in Example 1 and then genetically engineered to contain the hygromycin resistance gene (WO96/15238). The cells were cryopreserved as described earlier. Six months after cryopreservation, the cells were thawed and autologous reinfusion performed. The presence of DNA encoding the hygromycin resistance gene was analyzed by PCR at three-monthly intervals. The experiment shows the continuing presence of hygromycin-resistance gene-containing cells.

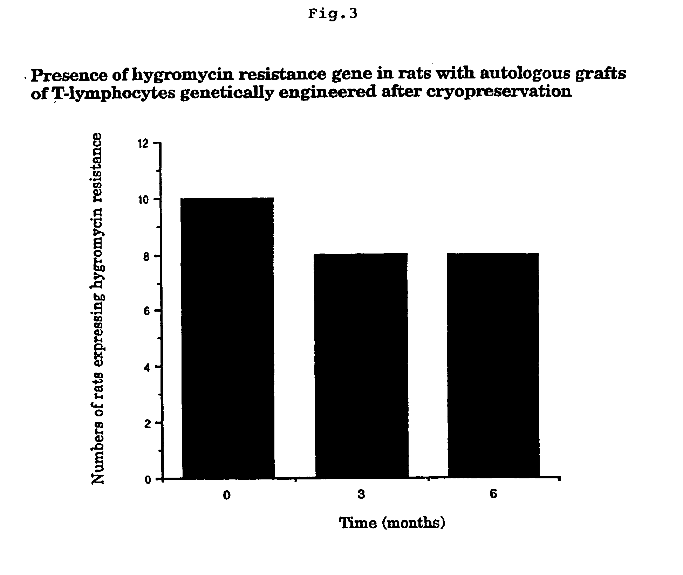

Figure 3 is a histogram showing the results of an identical study to that described in Figure 2, except that the genetic engineering step was performed after, rather than before the cryopreservation stage. The presence of DNA encoding the hygromycin resistance gene was analyzed by PCR at three-monthly intervals. Similar results to those found with a pre-preservation engineering step were obtained, indicating the prolonged survival of the cells after return to the host.

### Example 1

### Survival of infection through prospective autologous grafting of frozen stored donor cells

### (i) Samples taken

Both male and female Wistar rats were used in this study. A number of standard procedures were employed to extract either marrow samples or peripheral blood samples (see below). Reference to these procedures can be found in human or animal surgical texts such as that by Waynforth and Flecknell (Experimental and Surgical Technique in the Rat, 2nd Edition, Academic Press, 1992).

### (ii) Methods of sampling

In brief, animals were anaesthetised with chloroform and anaesthesia was maintained with halothane. Bone marrow cells and/or blood cells were sampled using standard procedures. All sampling was performed under anaesthesia. Blood was sampled by cardiac puncture, or by exposure of the jugular vein followed by blood extraction therefrom. Marrow was extracted from the femur after a hind leg amputation; and from the tibia and fibula of the amputated hind leg. (Practical Immunology, 3rd Edition, Blackwell Scientific Publications, 1989). For marrow sampling the rat femur and/or tibia was exposed and bone marrow cells removed using disposable bone aspirating needle(s) or an Islam bone marrow harvesting needle, or equivalent thereof, for rats. Several areas of the bone were sampled so as to provide an adequate harvest of marrow cells for future needs. Alternatively, a rib biopsy was taken which was particularly advantageous for the sampling of bone cells of the CFU-F (colony forming units-fibroblast) type. For humans, the iliac crest is usually sampled.

### (iii) Marrow cell preparation

Once obtained, the marrow cells were suspended in culture medium and separated from fatty materials essentially as described previously for human cell sampling (Bone 22(1998)7). The resulting cell suspension was transferred to a universal container and allowed to stand undisturbed for 10 minutes (min), after which time fat deposits that had floated to the top were removed. The marrow-derived cells were transferred to a centrifuge tube and spun at 100 x gravity (g) for 5 min to harvest the cells. The medium and fat deposits were again removed and the cell pellet resuspended in 5 ml of fresh culture medium. The resuspended cells were loaded onto a 70% Percoll gradient which was centrifuged at 460 g for 15 min. Following centrifugation, the top 25% of the gradient volume, which contained the required marrow cells, was removed. To this suspension an equal volume of fresh medium was added and the suspension centrifuged at 100 g for 10 min. The resulting cell pellet was resuspended in fresh medium and a single cell solution obtained by passing the cells through a 19-gauge needle several times. The number of viable cells was then determined by trypan blue (1% w/v) exclusion.

Alternatively, no separation of the fat cells from the sampled marrow cells was carried out, and the entire sample was prepared for dormancy. Both haematopoietically-derived and mesenchymally-derived tissues could be obtained by marrow sampling such that, in addition to cells of the immune system, cells capable of giving rise to osteoblasts, chondroblasts, myoblasts, fibroblasts and/or adipocytes could be also obtained. The mesenchymal cells can be separated, for example, by taking advantage of their adherent properties. Placing the sampled cells on standard tissue culture plasticware, for varying lengths of time, leads to adherence of the mesenchymal cell population to the plastic, leaving the haematopoietic cells in suspension. The different cell types can be then physically separated by pouring off the supernatant.

All of the above procedures are well known to the man skilled in the art.

### (iv) Peripheral blood sampling

Adequate samples of mononuclear cells (white cells) were obtained, alternatively, by peripheral blood sampling. It is well recognised that haematopoietic stem cells, progenitors of T-lymphocytes and mature T-lymphocytes reside in peripheral blood which makes peripheral blood mononuclear cells suitable for transplantation.

Peripheral blood was also sampled from rats given an intraperitoneal injection/s of either granulocyte colony stimulating factor (G-CSF) or granulocyte macrophage colony stimulating factor (GM-CSF) or haemopoietin for periods up to 96 hours prior to sampling. The peripheral blood mononuclear cells were sampled 1 to 4 days after G-CSF/GM-CSF administration. G-CSF and GM-CSF have been shown to increase the peripheral blood mononuclear cell population which comprises haematopoietic T-lymphocytes and their progenitors and stem cells, as well as other blood cells. This approach, therefore, helps to increase, *in vivo*, the abundance of cells required for subsequent transplantation prior to their removal from the body. The use of agents such as G-CSF, GM-CSF, haemopoietin or combinations thereof 3-4 days prior to sampling of blood by apheresis is a method currently used to obtain peripheral blood stem cells for human therapy and may be used in the invention (Stem Cells 15(1997)9).

### (v) Isolation and storage of sampled cells

Peripheral blood from either non-treated or GM-CSF/G-CSF-treated rats was taken, and white cells prepared directly using standard procedures. In short, blood samples were placed in heparinised tubes and high purity lymphocyte preparations obtained by differential centrifugation on a density gradient. After centrifugation, the white cell - containing buffy coat (white cell band), which was clearly visible, was removed to a fresh cryopreservation container (Practical Immunology, 3rd Edition, Blackwell Scientific Publications, 1989).

Alternatively, the marrow cell samples (either non-separated samples, or samples separated into different cell types - e.g. fat/non-fat, mesenchymal/haematopoietic cells) collected were placed in fresh cryopreservation containers.

To the blood or marrow samples autologous plasma containing 20% v/v DMSO (or variations of DMSO volume from 5-50%) was added to a final volume that brought the DMSO concentration to approximately 8.25 % (or variations of DMSO volume from 8-50%). The samples were then refrigerated and slowly frozen so as to lose approximately one degree Celsius every 1-2 min until they reached approximately minus 50°C. They were then transferred to gas/liquid-phase nitrogen/helium, or gas and/or liquid nitrogen followed by gas and/or liquid helium for long term storage at approximately minus 196°/269°C until required.

Rats sampled were given several weeks to recover prior to undergoing further treatment(s).

### (vi) Replenishment of the immune system

One group of 10 sampled rats received an ablative dose (8 Gy) of whole body irradiation so as to destroy radiation-sensitive cell populations. The radiation dose given has been shown to compromise the immune system which is highly radiation-sensitive, such that animals die readily from infection soon after treatment (Practical Immunology, 3rd Edition, Blackwell Scientific Publications, 1989). Removal of the thymus may have a similar effect.

Marrow cells or white cells were thawed from frozen, with gentle agitation of the cryovial in a beaker of 37°C water. Medium was then added to dilute the DMSO eg. 10-fold, and the cells gently pelleted by centrifugation at 400g. The cells were resuspended in a small volume of autologous plasma before being returned to the animal by infusion.

Of the 10 irradiated animals, 5 were given autologous grafts two weeks after irradiation, and all rats returned to the animal unit's main housing facility. Within three months the 5 non-autografted rats had died from infection, but the 5 autografted rats all survived the following six months of the study (see Figure 1).

### Example 2

### Autologous grafting of genetically engineered stored frozen donor lymphocytes

Both male and female Wistar rats were used in the study and marrow cells and/or peripheral blood mononuclear cell samples were obtained as described in Example 1. The mononuclear cells were genetically engineered to express the a and b chains of the T-cell receptor which, when combined, recognised the Mage 1 tumour antigen. The genetic construction also provided hygromycin resistance to the engineered cells and is described further in WO96/15238 - Targeted T-lymphocytes, incorporated herein.

Samples of the engineered cells were then cryopreserved for periods up to six months before being revitalised/revived as described in Example 1 and used as autologous grafts. Rats with autologous grafts were sacrificed at various times after grafting, and their blood ex-sanguinated by heart puncture. Genomic DNA encoding the hygromycin resistance gene, present in the engineered cells only, was detected by the polymerase chain reaction (PCR) as described previously (PCR A Practical Approach, IRL Press, 1991). In brief, peripheral blood mononuclear cells were isolated by differential centrifugation on a density gradient. The buffy coat was separated and genomic DNA prepared by phenol/ chloroform extraction followed by ethanol precipitation and resuspension in sterile water (Sambrook et al., Molecular Cloning. A Laboratory Manual, Vols. 1-3, Cold Spring Harbour Laboratory Press, 1989). The genomic DNA was PCR amplified in the presence of oligonucleotide primers designed to recognise a 272 base pair sequence of the hygromycin resistance gene (see McPherson et al., PCR. A Practical Approach, IRL Press, 1993 for PCR conditions). Hygromycin primers detecting hygromycin gene sequence of size 1.023 kb were as follows: GAATTCAGCGAGAGCCTGAC (left primer 5'-3') GATGTTGGCGACCTCGTATT (right primer 5'-3') A sample of the PCR product was electrophoresed through a 4% agarose gel and the 272 base pair fragment of the hygromycin resistance gene identified by UV transillumination after staining the gel with ethidium bromide. The ability to identify the hygromycin gene in the rat blood samples over time is provided in Figure 2.

### Example 3

### Genetic engineering and autologous grafting of stored frozen donor lymphocytes

Example 3 was performed as for Example 2, except that the cells for autologous grafting were cryopreserved prior to genetic engineering. Samples were thawed as described in Example 1 prior to autografting. Results of the maintenance of gene expression in the autograft over time is given in Figure 3.

### Example 4

### Syngeneic and autologous grafting of marrow stromal cells in young and aged rats

An inbred strain of Sprague Dawley rats was used for these studies. Suspensions of bone marrow cells (2x10⁶ cells per ml) were prepared from rib (also femur) biopsies taken from young rats (8 weeks of age) and aged rats (60 weeks of age). Cell samples were centrifuged at 400 X gravity and the resulting cell pellet(s) resuspended in 10% DMSO in autologous plasma followed by cryopreservation in liquid nitrogen and/or followed by liquid helium.

Cell samples were revitalised from 3 months to 2 years after cryopreservation and suspended in culture medium at 2 x 10⁶ cells/ml. To each sample suspension a single porous hydroxyapatite disc was added and left for 24 hours to allow cells to adhere to it - producing a cell/HA composite. The composites were then implanted subcutaneously as either autogenous or syngeneic grafts. The grafts were removed after 8 weeks and subjected to histological analysis, and osteocalcin and alkaline phosphatase measurements.

The experimental groups were as follows
(1) Marrow cells sampled from young rats aged 8 weeks were incubated with porous hydroxyapatite (HA) for 24 hours to form a marrow cell/HA composite. The composites were then either (a) autogenously grafted or (b) syngeneically grafted to rats of the same age or (c) rats of 60 weeks of age, or (d) rats of 104 weeks of age.
(2) Marrow cells sampled from old rats aged 60 weeks were incubated with porous hydroxyapatite for 24 hours to form a marrow cell/HA composite. The composites were then either (a) autogenously grafted or (b) syngeneically grafted to rats of 8 weeks of age or (c) syngeneically grafted to rats of 60 weeks of age, or (d) syngeneically grafted to rats of 104 weeks of age.
(3) Marrow cells sampled from young rats aged 8 weeks were cryopreserved as described. After 96 weeks cryopreservation, the cells were revitalised and incubated with porous hydroxyapatite for 24 hours to form a marrow cell/HA composite. The composites were then either (a) autogenously grafted (the sampled rats being 104 weeks of age) or (b) syngeneically grafted to rats of 104 weeks of age or (c) syngeneically grafted to rats of 8 weeks of age.
(4) Marrow cells sampled from old rats aged 60 weeks were cryopreserved as described. After 44 weeks cryopreservation, the cells were revitalised and incubated with porous hydroxyapatite for 24 hours to form a marrow cell/HA composite. The composites were then either (a) autogenously grafted (the sampled rats being 104 weeks of age) or (b) syngeneically grafted to rats of 104 weeks of age or (c) syngeneically grafted to rats of 8 weeks of age.

### Results

Qualitative histological analysis demonstrated a clear difference between the ability of young and old marrow cells to induce new bone formation in either autogenous or syngeneic grafts. 40% of old marrow cell/HA composites showed no bone formation when grafted to either young or old rats, whereas bone was formed in all composites comprising young marrow cells. The result was identical for composites where the marrow cells had been cryopreserved and revitalised prior to grafting.

Differences in osteocalcin expression and alkaline phosphatase activity between composites formed from young and old marrow cells was highly significant. On average, osteocalcin expression was 8-10 fold higher in composites containing young cells when compared to composites containing old cells. The 8-10 ratio of osteocalcin expressed between composites comprising young compared to composites comprising old cells did not vary significantly due to cryopreservation and revitalisation, or because of syngeneic rather than autologous grafting.

Alkaline phosphatase activity was also seen to vary between composites comprising either young or old cells. Composites comprising young cells had 4-6 fold the alkaline phosphatase activity of old cell composites. Similar to the osteocalcin study, no significant change to this ratio was brought about by cryopreservation of cells prior to forming the composites; or from syngeneic grafting rather than autografting.

Table 1 below discloses the ratios of osteocalcin expression seen between the different groups:

**Table 1**

| Group x/Group y | Ratio |
|---|---|
| 1a/1b | 1.3 |
| 1a/1c | 1.4 |
| 1a/1d | 1.5 |
| 2a/2b | 0.9 |
| 2a/2c | 1.4 |
| 2a/2d | 1.6 |
| 3a/3b | 1.0 |
| 3a/3c | 1.0 |
| 4a/4b | 1.3 |
| 4a/4c | 0.9 |
| 1a/2a | 9.7 |
| 3a/4a | 9.8 |
| 1a/3a | 1.4 |
| 2a/4a | 1.4 |

Table 2 below discloses the ratios of alkaline phosphatase expression seen between the different groups:

**Table 2**

| Group x/Group y | Ratio |
|---|---|
| 1a/1b | 1.1 |
| 1a/1c | 1.3 |
| 1a/1d | 1.3 |
| 2a/2b | 1.3 |
| 2a/2c | 1.5 |
| 2a/2d | 1.8 |
| 3a/3b | 1.1 |
| 3a/3c | 0.9 |
| 4a/4b | 1.3 |
| 4a/4c | 0.9 |
| 1a/2a | 5.8 |
| 3a/4a | 5.9 |
| 1a/3a | 1.1 |
| 2a/4a | 1.1 |

Similar differences in bone forming ability, and in bone gla protein and alkaline phosphatase activity, between marrow cells from young and old rats has been reported independently by Inoue et al (1997) using syngeneic rats. However, Inoue et al have not reported the effects of cryopreservation on the osteogenic ability of young and old cells, and whether this clear difference in young and old cell osteogenic ability holds true for autogenous grafts.

### Example 5

### Autologous grafting of neural cells to adult rats

Adult rats at 3 months of age were placed in a stereotaxic frame, and the area of skull overlying the lateral ventricle at the level of bregma was removed. A blunt-ended, sterile glass micropipette (ID = 100 mm) was inserted into the brain 1.4 mm lateral to the midline. The pipette was lowered into the dorsal part of the lateral ventricle at which point, with controlled suction, a small amount of cerebrospinal fluid (CSF) was withdrawn. Still with controlled suction, the pipette was further lowered so that it passed through the ventricular, subventricular and other layers of neural tissue situated each side of the lateral ventricle. A suspension of tissue, cells and CSF collected in the large diameter part of the pipette. At the end of the biopsy, the suspension was triturated (sometimes with prior enzymatic digestion with eg. trypsin) and ejected into a small tissue culture flask containing medium. Such medium comprised a mixture of Dulbecco's modified Eagle's medium and Ham's F12 (50/50 v/v) supplemented with L-glutamine (2mM), penicillin:streptomycin (100 IU/ml: 10 mg/ml) and modified stock solution (PNAS USA 76(1979)514; J Neurophysiol 40(1981)1132) containing 10 ng/ml epidermal growth factor (EGF), 5 ng/ml basic fibroblast growth factor (FGF), or the like. Sometimes, transmembrane co-culture with replicative neural cells, or conditioned medium from such cells was also used to support the survival of the newly-dissociated adult cells.

The clusters of replicating cells were expanded, and "passaged" by sectioning into. 4 - 6 parts followed by replating. This allowed prolonged expansion. Cell clusters, or mechanically dissociated cells derived from them, were frozen either at this stage, or after differentiation (vide infra), in medium containing 10% DMSO and using conventional methods. They were then placed in gas/liquid phase nitrogen followed by prolonged storage in gas/liquid phase helium for periods of up to or including one year.

Thawed frozen replicative cells, or cells from dissociated replicative clusters were replated in roller tubes and allowed to differentiate in the same medium, but without EGF, for the next few days. Some cells were differentiated in the presence of medium conditioned with confluent (but still replicative) striatal cells to provide both support and differentiation-directing/inducing factors. Thereafter, the resulting differentiated cell clusters (approximately 1 million cells) were injected into the original donor rats lesioned 10 days earlier unilaterally by intrastriatal injection of ibotenic acid. Alternatively, the cells were frozen as described above, and then thawed and injected into the lesioned striatum. Three months later the animals were fixed by perfusion, and the grafts analyzed by immunohistochemistry.

Surviving transplants were found in all grafted animals. The implants were well integrated into the host striatum, although they could still be clearly demarcated as an area through which myelinated fibre bundles mostly failed to grow. The proportion of surviving grafts was not affected by whether the donor cells had been frozen at any stage. Indeed, those animals with cells that had been through a freezing procedure possessed larger grafts than non-frozen cells, and the expression of neurofilament in such grafted cells also appeared to be more intense and extensive. Similarly, some GFAP expression could be seen in the graft.

### Example 6

### Effects of storage temperature on cell viability after one year

Duplicate aliquots of the cells of the type shown were frozen by methods described in the text, and at the end of 1 hour of cooling at 1°C per minute were placed in liquid nitrogen for 1 hour. At that time, one of each pair of aliquots was thawed by the methods described in the text, and analyzed for cell viability using ethidium bromide exclusion/acridine orange incorporation (Brain Res 331 (1985) 251). The other aliquot was placed in liquid helium for approximately one year, and then thawed and cell viability assessed by the same process. The results are shown in Table 3 below. Figures are the means and SEM of 6 sample pairs, and are expressed as a proportion of the cells surviving after 1 hour in liquid nitrogen. It will be seen that the lower temperature can lead to a small but significant enhancement of the viability of the cells in the long term.

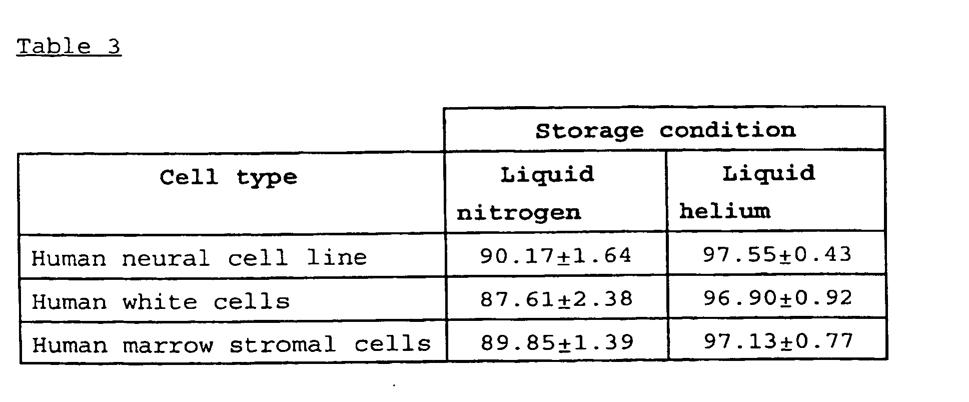

### Example 7

### Effects of storage temperature on cell viability after two years

The "one year" study of Example 6 was repeated, storing an aliquot of cells in liquid helium for 2 years. The cells were then thawed and cell viability was assessed as described in Example 6. The results are shown in Table 4. Figures are the means and SEM of 6 sample pairs, and are expressed as a proportion of the cells surviving after 1 hour in liquid nitrogen. It will be seen that, after two years storage similarly good viability results may be obtained.

**Table 4**

| **Cell type** | **Liquid nitrogen** | **Liquid helium** |
|---|---|---|
| Human neural cell line | 89.28±2.51 | 97.08±1.32 |
| Human white cells | 88.19±0.96 | 96.98±1.41 |
| Human marrow stromal cells | 86.20±1.67 | 97.56±0.93 |

## Claims

1. Use of a host cell population of mature, healthy lymphocyte cells obtained from blood of a non-diseased host organism for the preparation of a cell composition for use in subsequent autologous therapeutic transplantation therapy of a disease or disorder of said host organism, wherein the cells are obtained from the host organism before the disease or disorder develops or manifests itself.

2. A use as claimed in claim 1, wherein said host cells are harvested from said host organism at a stage when there is no direct prediction, suggestion or suspicion that said disease or disorder may develop.

3. A use as claimed in claim 1 or claim 2, wherein said host organism is a human.

4. A use as claimed in any one of claims 1 to 3, wherein said host organism is juvenile, adolescent or adult, at the time the cells are obtained.

5. A use as claimed in any one of claims 1 to 4, wherein the immune system of said host organism is mature, at the time the cells are obtained.

6. A use as claimed in any one of claims 1 to 5, wherein the immune system of said host organism is uncompromised, at the time the cells are obtained.

7. A use as claimed in any one of claims 1 to 6, wherein said lymphocyte cells are T-lymphocyte cells.

8. A use as claimed in any one of claims 1 to 7, wherein said therapy is therapy of a chronic condition.

9. A use as claimed in any one of claims 1 to7, wherein said therapy is cancer therapy.

10. A use as claimed in any one of claims I to 7, wherein said therapy is for HIV infection or AIDS.

11. A use as claimed in claim 10, wherein said host cell population comprises CD4⁺ cells.

12. A use as claimed in any one of claims 1 to 11, wherein said host cell population is maintained in a state of dormancy.

13. A use as claimed in any one of claims 1 to 12, wherein said host cell population comprises a genetically modified cell

14. A use as claimed in any one of claims 1 to 13 wherein, after removal from the host and before transplantation, said host cell population is stored by freezing the cells.

15. A use as claimed in claim 14, wherein the host cell population is frozen to about -269°C or below.

16. A method of making and/or maintaining lymphocyte cells dormant, said method comprising freezing said lymphocyte cells to a temperature at or below -269°C.

17. A method as claimed in claim 16 wherein said lymphocyte cells are obtained from blood.

18. A method as claimed in claim 16 or claim 17 wherein said lymphocyte cells are T-lymphocyte cells.

## Patentansprüche

1. Verwendung einer Wirtszellpopulation von ausgereiften, gesunden Lymphozytenzellen, erhalten vom Blut eines nicht erkrankten Wirtsorganismus, zur Herstellung einer Zellzusammensetzung zur Verwendung bei einer nachfolgenden autologen therapeutischen Transplantationstherapie einer Krankheit oder Erkrankung des Wirtsorganismus, worin die Zellen vom Wirtsorganismus erhalten werden, ehe sich die Krankheit oder Erkrankung entwickelt oder manifestiert.

2. Verwendung nach Anspruch 1, worin die Wirtszellen von dem Wirtsorganismus in einem Stadium geerntet werden, in dem es keine direkte Vorhersage, keine Hinweis oder Verdacht gibt, dass sich die Krankheit oder Erkrankung entwickeln kann.

3. Verwendung nach Anspruch 1 oder Anspruch 2, worin der Wirtsorganismus ein Mensch ist.

4. Verwendung nach einem der Ansprüche 1 bis 3, worin der Wirtsorganismus zu dem Zeitpunkt, an dem die Zellen erhalten werden, juvenil, adoleszent oder adult ist.

5. Verwendung nach einem der Ansprüche 1 bis 4, worin das Immunsystem des Wirtsorganismus zu dem Zeitpunkt, an dem die Zellen erhalten werden, ausgereift ist.

6. Verwendung nach einem der Ansprüche 1 bis 5, worin das das Immunsystem des Wirtsorganismus zu dem Zeitpunkt, an dem die Zellen erhalten werden, nicht beeinträchtigt ist.

7. Verwendung nach einem der Ansprüche 1 bis 6, worin die Lymphozytenzellen T-Lymphozytenzellen sind.

8. Verwendung nach einem der Ansprüche 1 bis 7, worin die Therapie eine Therapie eines chronischen Zustands ist.

9. Verwendung nach einem der Ansprüche 1 bis 7, worin die Therapie eine Krebstherapie ist.

10. Verwendung nach einem der Ansprüche 1 bis 7, worin die Therapie für eine HIV-Infektion oder für AIDS ist.

11. Verwendung nach Anspruch 10, worin die Wirtszellpopulation CD4+-Zellen umfasst.

12. Verwendung nach einem der Ansprüche 1 bis 11, worin die Wirtszellpopulation in einem Ruhezustand gehalten wird.

13. Verwendung nach einem der Ansprüche 1 bis 12, worin die Wirtszellpopulation eine genetisch modifizierte Zelle umfasst.

14. Verwendung nach einem der Ansprüche 1 bis 13, worin die Wirtszellpopulation nach dem Entfernen aus dem Wirt und vor einer Transplantation durch Einfrieren der Zellen gelagert wird.

15. Verwendung nach Anspruch 14, worin die Wirtszellpopulation auf etwa -269°C oder weniger eingefroren wird.

16. Verfahren zur Herstellung und/oder zum Halten von Lymphozytenzellen im Ruhezustand, wobei das Verfahren Einfrieren der Lymphozytenzellen auf eine Temperatur von -269°C oder weniger umfasst.

17. Verfahren nach Anspruch 16, worin die Lymphozytenzellen aus Blut erhalten werden.

18. Verfahren nach Anspruch 16 oder Anspruch 17, worin die Lymphozytenzellen T-Lymphozytenzellen sind.

## Revendications

1. Utilisation d'une population de cellules hôtes de cellules lymphocytaires matures et saines obtenues du sang d'un organisme hôte non malade pour la préparation d'une composition cellulaire destinée à être utilisée dans une thérapie par transplantation thérapeutique autologue subséquente d'une maladie ou d'un trouble dudit organisme hôte, dans laquelle les cellules sont obtenues de l'organisme hôte avant que la maladie elle-même ou le trouble lui-même ne se développe ou ne se manifeste.

2. Utilisation telle que revendiquée dans la revendication 1, dans laquelle lesdites cellules hôtes sont récoltées dudit organisme hôte à un stade où il n'y a pas de prédiction, suggestion ou suspicion directe indiquant que ladite maladie ou ledit trouble peut se développer.

3. Utilisation telle que revendiquée dans la revendication 1 ou la revendication 2, dans laquelle ledit organisme hôte est un humain.

4. Utilisation telle que revendiquée dans l'une quelconque des revendications 1 à 3, dans laquelle ledit organisme hôte est enfant, adolescent ou adulte, au moment où les cellules sont obtenues.

5. Utilisation telle que revendiquée dans l'une quelconque des revendications 1 à 4, dans laquelle le système immunitaire dudit organisme hôte est mature, au moment où les cellules sont obtenues.

6. Utilisation telle que revendiquée dans l'une quelconque des revendications 1 à 5, dans laquelle le système immunitaire dudit organisme hôte n'est pas compromis, au moment où les cellules sont obtenues.

7. Utilisation telle que revendiquée dans l'une quelconque des revendications 1 à 6, dans laquelle lesdites cellules lymphocytaires sont des lymphocytes T.

8. Utilisation telle que revendiquée dans l'une quelconque des revendications 1 à 7, dans laquelle ladite thérapie est une thérapie d'un état chronique.

9. Utilisation telle que revendiquée dans l'une quelconque des revendications 1 à 7, dans laquelle ladite thérapie est une thérapie du le cancer.

10. Utilisation telle que revendiquée dans l'une quelconque des revendications 1 à 7, dans laquelle ladite thérapie est une thérapie d'une infection par le VIH ou du le SIDA.

11. Utilisation telle que revendiquée dans la revendication 10, dans laquelle ladite population de cellules hôtes comprend des cellules CD4⁺.

12. Utilisation telle que revendiquée dans l'une quelconque des revendications 1 à 11, dans laquelle ladite population de cellules hôtes est maintenue dans un état de dormance.

13. Utilisation telle que revendiquée dans l'une quelconque des revendications 1 à 12, dans laquelle ladite population de cellules hôtes comprend une cellule génétiquement modifiée.

14. Utilisation telle que revendiquée dans l'une quelconque des revendications 1 à 13, dans laquelle, après le prélèvement chez l'hôte et avant la transplantation, ladite population de cellules hôtes est stockée par congélation des cellules.

15. Utilisation telle que revendiquée dans la revendication 14, dans laquelle la population de cellules hôtes est congelée à environ -269°C ou moins.

16. Procédé de fabrication et/ou de maintien de cellules lymphocytaires dormantes, ledit procédé comprenant la congélation desdites cellules lymphocytaires à une température de, ou inférieure à, -269°C.

17. Procédé tel que revendiqué dans la revendication 16, dans lequel lesdites cellules lymphocytaires sont obtenues du sang.

18. Procédé tel que revendiqué dans la revendication 16 ou la revendication 17, dans lequel lesdites cellules lymphocytaires sont des lymphocytes T.
